# EUROPEAN PATENT APPLICATION

(11) **EP 1 004 300 A1**
(43) Date of publication of application: **31.05.2000**
(21) Application number: 98929819.5
(22) Date of filing: 03.07.1998
(51) Int. Cl.: A61K 31/13, A61K 31/195, A23K 1/16

(54) **ANION REGULATOR FOR RUMINANTS**

(30) Priority: 08.07.1997 JP 18216197
(71) Applicant: Ajinomoto Co., Inc., Tokyo 104-0031 (JP)
(72) Inventor: USUI, Naoki, Ajinomoto Co., Inc., Tokyo 104-0031 (JP); KOBAYASHI, Hisamine, Ajinomoto Co., Inc., Kawasaki-shi, Kanagawa-ken 210-0801 (JP); CHINO, Masao, Ajinomoto Co., Inc., Tokyo 104-0031 (JP); NAKAMURA, Yoshihiro, Ajinomoto Co., Inc., Tokyo 104-0031 (JP); TAKEMOTO, Tadashi, Ajinomoto Co., Inc., Kawasaki-shi, Kanagawa-ken 210-0801 (JP)
(74) Representative: Strehl Schübel-Hopf & Partner
(86) International application number: JP9803000
(87) International publication number: WO9902144

(57) **Abstract**

Here is disclosed an anion regulator for ruminants comprising, as the active ingredient, the neutral hydrochloride of an amino compound, such as a basic amino acid monohydrochloride, which anion regulator shows good palatability to ruminants, does not reduce feed intake by mother animals before parturition, and is excellent in anion regulatory function.

## Description

### (Technical Field)

This invention relates to an anion regulator for ruminants. More particularly, this invention relates to an anion regulator for ruminants comprising, as the active ingredient, a neutral hydrochloride of an amino compound such as a basic amino acid, as well as to a ruminant feed added with such anion regulator.

### (Background Art)

Production of milk by dairy cattle has been significantly improved as a result of long term studies. Improvements in handling and feeding techniques and the introduction of cattle with high lactation ability have resulted in surprising levels of average milk production per head, exceeding 10,000 liters per a year. Even still now, however, much economic loss occurs as a result of the occurrence of various diseases contracted often around, or before and after, parturition. For example, the investigation in 100 fields, conducted by the Michigan State University in 1995, indicates that diseases such as ketosis, abomasum displacement, retained placenta and milk fever, occur with incidences of as much as 12%, 11%, 12% and 8%, respectively (Dairy Japan for October 1996, p.40). These diseases lead not only to decreased production of milk but also to reduced breeding efficiency and losses such as death, in severe cases. Therefore, overcoming these diseases is an important matter to be solved in dairy farming at present.

It has been pointed out that rapid metabolic changes caused by milk production immediately after parturition are extremely involved in these diseases. As much as 23 g of calcium is contained in about 10 liters of average colostrum of dairy cattle. This calcium amounts to as many as eight(8) to ten(10) times the amount of calcium in the blood. In healthy dairy cattle, the colostric calcium is rapidly supplied through the blood calcium from the bone. For some reason, however, if the rate of calcium supply from the bone is slow, milk fever is generated due to a temporal decrease in the blood calcium level. Further, decreases in the blood calcium level also cause hypofunction of the smooth muscle contractions of the uterus and digestive tract, resulting in retained placenta and dystocia. In addition, this low level of blood calcium causes abomasum displacement due to hypofunction of the digestive tract and ketosis due to decreased feed intake. In other words, hypocalcemia causes various symptoms.

For prevention of these diseases it is effective to increase the calcium transfer from the bone prior to parturition. To cause such increase, a method of feeding low calcium feed before parturition, and another method of feeding feed having an ion balance more in anions of Cl and S than in cations of K and Na, have been previously shown to be effective.

The former is the method to bring the artificial condition of a slight calcium deficiency prior to the production of the colostrum in order to stimulate calcium metabolism from the bone for the production of colostrum. However, alfalfa, which is an economical and highly nutritious feed, and therefore, currently much used, is rich in calcium content, and consequently is difficult to use. In fact, specific feeds are not very often prepared from raw feed materials with low calcium content, only for cattle immediately before the parturition.

Whereas, a method for regulating the ion balance of feed widely attracts attention. This is an attempt to promote calcium metabolism in the bone by feeding anion-rich feed for about 3 weeks just before the parturition, which, in turn, causes a slight acidosis in dairy cattle just before the parturition. For example, Block reported in J. Dairy Sci., (1984) 67: 2939, studies where feeds having different DCAD (Dietary Cation-Anion Difference) values (unit: meq/kg-dry matter), which are defined by an ion balance of (Na + K)-(Cl + S), were fed to two groups of dairy cattle before parturition, and the incidence of diseases related to hypocalcemia immediately after parturition was observed. The results indicated that, in a group fed with a positive DCAD value feed, approximately 50% of the cattle showed symptoms, whereas in a group fed with a negative DCAD value feed, no symptoms were observed. Oetzel et al. also reported in J. Dairy Sci., (1988) 71: 3302, that although the conditions were different from those above, animals fed with a negative DCAD value feed showed similarly a decreased incidence of diseases due to hypocalcemia. Nowadays, it has got common to supply feed having negative DCAD values to dairy cattle before parturition, generally as an effective means for reducing diseases immediately after parturition. The action of such feed supplies might be due to the fact that feed supplies with negative DCAD values cause slight metabolic acidosis, which, in turn, results in increased activities of parathyroid hormone (PTH) and active vitamin D, whereby calcium metabolism in the bone and absorption of calcium from feed are promoted.

It is an anion regulator that can be used to regulate the ion balance (DCAD value) in an easy way. For promoting calcium metabolism, it is recommended that the DCAD value should be regulated to between -100 and -150 meq/kg-dry feed matter for approximately 3 weeks immediately before parturition. However, recently, the potassium content in raw feed materials has been increasing. Consequently, it is almost impossible to prepare feeds with a low DCAD value from combinations of common raw feed materials. Further, it is said that dairy cattle should be appropriately fed with positive DCAD value feeds during the lactating period. In such circumstances, it is not practical to prepare specific feeds having a negative DCAD value, only for a relatively small number of dairy cattle just before parturition. Consequently, an anion regulator which can conveniently reduce DCAD values, has got required. In general, if such an anion regulator is added to feed, the DCAD value of the feed can simply be regulated to a negative value.

Conventionally, inorganic salts such as NH₄Cl, MgSO₄ or CaSO₄ have been used as anion regulators. Combinations of these salts have been used due to the limitations on the physiological requirements of Mg and Ca, and toxicity of each salt [HOARD'S DAIRYMAN (1997) 103: 212 (Japanese version)]. Dairy farmers formulate feeds, using various raw feed materials in combination, in consideration of regional, seasonal and economic conditions. Dairy farmers perform general analysis of the formulated feed, and as a result of the analysis, deficient amounts of Mg, S and others are supplied by using anion regulators comprising such salts one after the other. As a result of such procedures, the feed supply to be used before parturition is finally prepared.

A preparation using inorganic salts such as NH₄Cl, MgSO₄ and CaSO₄ as the anion regulator does, however, result sometimes in decreased intake of feed added with such preparation, because such inorganic salts per se are less palatable to cattle [HOARD'S DAIRYMAN (1990), Apr., 344 (Japanese version)]. The stage before parturition is an especially important preparative period for the stage of lactation after parturition. Decreases in feed intake during such an important stage results not only in reduced efficiency of milk production but also in reduced breeding efficiency during the next stage. These, in turn, then result in large economic losses.

Consequently, an anion regulator which has good palatability, does not reduce feed intake by the mother cattle before parturition and has an excellent anion regulating activity, is strongly needed.

### (Disclosure of the Invention)

The present inventors have found, as a result of extensive studies in order to solve the above problems, that a neutral hydrochloride of an amino compound is effective as an anion regulator which satisfies the above desired specificity, and completed the present invention based on such findings.

Accordingly, the present invention relates to an anion regulator for ruminants comprising, as the active ingredient, a neutral hydrochloride of an amino compound. It also relates to feeds for ruminants added with such an anion regulator.

The present invention will be described in detail as follows.

The active ingredient of an anion regulator for ruminants of the present invention is a neutral hydrochloride of an amino compound. "The neutral hydrochloride of an amino compound" referred to herein, in relation to the present invention will be described. For example, in cases of basic amino acids such as lysine or ornithine having two amino groups per one molecule, out of their mono- and di- hydrochlorides, the monohydrochlorides (or monohydrochloric acid salts) are neutral hydrochlorides, according to the present invention. In such a basic amino acid, out of the two amino groups in one molecule thereof, one amino group constitutes the inner salt structure along with the carboxyl group in the said basic amino acid molecule, and therefore, an basic amino acid is a monoacidic base as the whole molecule. The said monoacidic base is added with one molecule of a monobasic acid, i.e., hydrochloric acid (to be exact, hydrogen chloride), to form an acid addition salt (hydrochloric acid addition salt), which is a monohydrochloride. The said acid addition salt has the composition of a neutral salt consisting of equivalent amounts of an acid and a base. Therefore, this is designated as a neutral hydrochloride. (As compared to the above, for example, lysine dihydrochloride is not a neutral hydrochloride, but is an acidic hydrochloride. In such dihydrochloride, the two amino groups of the lysine molecule are both constructed as acid addition salts with externally added hydrochloride, and the carboxyl group which originally existent in the lysine molecule forms a free carboxyl group.) Similarly, in cases of peptides or proteins, being amino compounds, hydrochloric acid addition salt in which one hydrochloric acid molecule is bound to each amino group which has the ability to form an acid addition salt with external acid, except for those amino groups which constitute inner salts with the carboxyl groups therein, is a neutral hydrochloride of peptide or protein as an amino compound, according to the present invention.

As is well known, ammonia with its one or more of the hydrogen atoms in the molecule having been replaced by hydrocarbon radical(s) are collectively designated as amines. The amines are grouped as primary amine (RNH₂), secondary amine (RR'NH), and tertiary amine (RR'R''N), according to the number of substituent(s) on the nitrogen atom. Those compounds having an amino group are designated as amino compounds. A compound in which all of the R (R' and R'') are alkyl group(s) (or having substituent(s)), is designated as an aliphatic amine, and a compound in which all of the R (R' and R'') groups or some of them are aromatic hydrocarbon radicals, is designated as an aromatic amine. An amine compound which has one nitrogen atom in the form. of an amino group or imino group in one molecule, is designated as a monoamine. Similarly, a compound having two such nitrogen atoms is called diamine (for example cadaverine), and a compound having multiple amino groups is generally called polyamine.

The amino compound according to the present invention is a compound defined by the above definition, and includes not only amino acids, but also proteins having the above designated amino groups and peptides as hydrolysates of the said proteins.

Neutral hydrochlorides of these amino compounds can be the active ingredient of the anion regulators for ruminants of the present invention. Examples of such neutral hydrochlorides include a monohydrochloride of a basic amino acid such as lysine, ornithine or arginine, an acid precipitation fraction of soybean protein precipitated with hydrochloric acid (hydrochloric acid is being added thereto), and an acid precipitation fraction of peptide separated from a protein hydrolysate with hydrochloric acid (hydrochloric acid is being added thereto). The chlorine ions of the hydrochloric acid, which are contained in these compounds, act as the Cl in the DCAD value mentioned previously.

The acidic hydrochloride of an amino compound, for example, lysine dihydrochloride or glutamic acid monohydrochloride, is not palatable to cattle. This maybe due to their strongly acidic nature, and they are not preferable as anion regulators for ruminants.

No specific difficulties are encountered in preparing an anion regulator for ruminants of the present invention, which comprises the neutral hydrochloride of an amino compound as the active ingredient. It can be easily prepared by using only the neutral salt of an amino compound, or by adding raw feed materials or feed additives commonly used in the field of feeds to the said neutral hydrochloride. The thus prepared product can be put into distribution channel in an appropriate packaging form as a powder or as any other formulation.

The anion regulator described above can be blended with a formulated feed or concentrated feed, and can be distributed in that form, i.e., as feed for ruminants which has been already mixed or added with the anion regulator.

The period for feeding the anion regulator for ruminant of the present invention may be approximately three weeks immediately prior to parturition, as mentioned above, which is effective to prevent hypocalcemia.

The amount of the anion regulator for ruminants which is effective for prevention of hypocalcemia can be determined, for example, by measuring the urinary pH of animals to which the anion regulator has been fed. Namely, the pH of the urine is observed to be between pH 6.5 - 5.5 with an appropriately administered amount of the anion regulator for ruminants. Refer to Example 3 below.

No special feeding method is necessary. Feeding can be performed by giving the feed to ruminants such as cattle, goats or sheep in the form of formulated feed or concentrated feed admixed with the anion regulator, or by feeding formulated feed, concentrated feed or other feeds, on which the anion regulator has been sprayed.

### (Brief Description of Drawing)

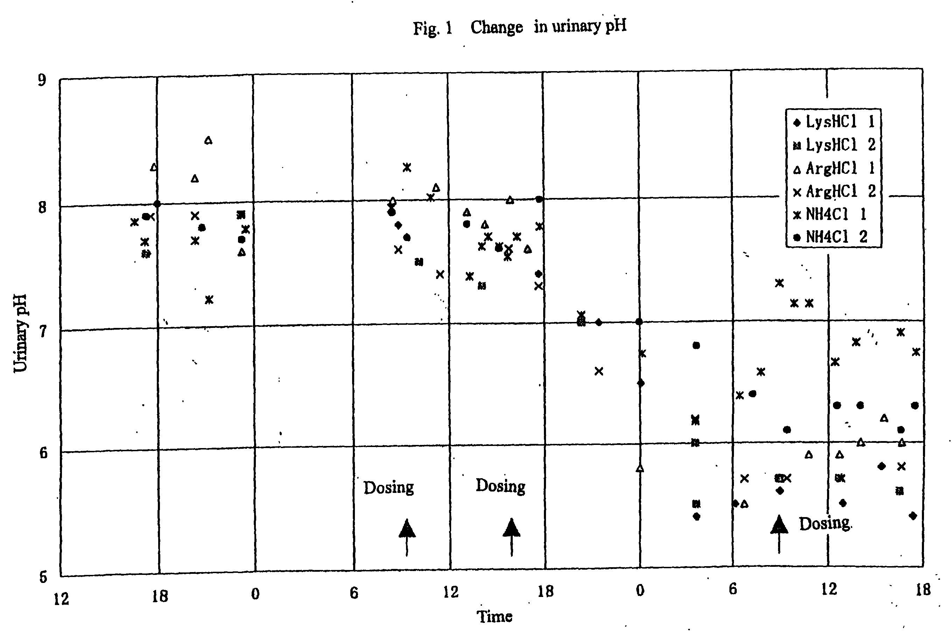

Fig. 1 shows changes in urinary pH in Example 3.

### (Best Mode for Carrying out the Invention)

The following examples will describe further the present invention, but are not to be construed as limiting the present invention. Any modification or design variation developed by referring to the gist of the present invention described heretofore or hereinafter is included within the technical scope of the present invention.

### Example 1 (Palatability test No. 1)

A palatability test was conducted using three(3) Holstein calves with a body weight of 200 kg (Calf 1 - 3). Feed composed of 1,100 g of concentrated feed ("Beef-fast", ex Itochu Feed Co.), 550 g of timothy-hay and 550 g of alfalfa hay cubes was fed twice a day at 9 and 16 o'clock for one week for adaptation.

16 g of NH₄Cl, 37 g of MgSO₄ · 7H₂O, 22 g of CaCl₂ · 2H₂O, 55 g of L-lysine monohydrochloride, 63 g of L-arginine monohydrochloride and 51 g of L-ornithine monohydrochloride were respectively measured to be in an amount equivalent to -300 meq, and each compound was admixed with 100 g of concentrated feed. All the mixed feeds were placed simultaneously at the time of feeding, using shallow trays in order to allow the calves to take in the feed freely. After 10 minutes of feeding, the remaining feeds were measured in terms of weight(%).

The initial amount of mixed feeds supplied was set as 100%. The remaining amount was deducted from the initial amount to determine the amount of intake. A minus figure for the amount of intake indicates that the palatability to the calves was not good and shows that the increased weight of a mixed feed supplied was due to mixing of saliva from the calf.

The results are shown in Table 1. As is understood from the table, lysine hydrochloride, arginine hydrochloride and ornithine hydrochloride showed better palatabilities than NH₄Cl, MgSO₄ · 7H₂O and CaCl₂ · 2H₂O.

**Table 1**

| Additive | Amount of intake (%) | | |
|---|---|---|---|
| | Calf 1 | Calf 2 | Calf 3 |
| NH₄Cl | 38 | 11 | 43 |
| MgSO₄ · 7H₂O | 35 | 14 | 50 |
| CaCl₂ · 2H₂O | 0 | 0 | -3 |
| L-lysine hydrochloride | 40 | 15 | 80 |
| L-arginine hydrochloride | 44 | 17 | 63 |
| L-ornithine hydrochloride | 65 | 15 | 52 |

### Example 2 (Palatability test No. 2)

This palatability test was conducted in the same way as in Example 1, but using three(3) Holstein milk cows (Cows 4 - 6), which were in their dry period. Their body weight was approximately 650 kg. A basal diet consisting of concentrated feed, timothy-hay and alfalfa hay cubes, was fed in an amount of the maintenance ration according to Japanese feeding standards.

32 g of NH₄Cl, 74 g of MgSO₄ · 7H₂O, 44 g of CaCl₂ · 2H₂O, 110 g of L-lysine monohydrochloride, 126 g of L-arginine monohydrochloride and 102 g of L-ornithine monohydrochloride were respectively measured to be in an amount equivalent to -600 meq, and each compound was admixed with 200 g of concentrated feed. All the said admixed feeds were placed in such way that each cow was allowed to select freely any one thereof at feeding time. Ten minutes later, the remaining amount of each feed was measured in terms of weight(%). The intake amounts in weight per cent were determined in the same way as in Example 1.

The results are shown in Table 2. As is understood from Table 2, lysine hydrochloride, arginine hydrochloride and ornithine hydrochloride showed better palatabilities than NH₄Cl, MgSO₄ · 7H₂O and CaCl₂ · 2H₂O.

**Table 2**

| Additive | Amount of intake (%) | | |
|---|---|---|---|
| | Cow 4 | Cow 5 | Cow 6 |
| NH₄Cl | 43 | 47 | 33 |
| MgSO₄ · 7H₂O | 55 | 15 | 24 |
| CaCl₂ · 2H₂O | 27 | -9 | 0 |
| L-lysine hydrochloride | 89 | 88 | 73 |
| L-arginine hydrochloride | 73 | 50 | 35 |
| L-ornithine hydrochloride | 55 | 70 | 40 |

### Example 3 (Test on anion regulating activity)

A simple method for determining the effectiveness of anion regulators has been shown to be the measurement of urinary pH. If a DCAD value is positive, the urinary pH is 7 - 8.5. It is also known that if an anion regulator is added, and as a result the feed is changed to show a negative DCAD value, the urinary pH of animals to which such feed has been fed is reduced and will show a value of 6.5 - 5.5 when the anion regulator has been added in an appropriate amount. Refer to HOARD'S DAIRYMAN (1995), Sep., p.634 (Japanese version).

In order to establish the usefulness of lysine hydrochloride and arginine hydrochloride as anion regulators, each compound was added to the feed, and the urinary pH of the cattle which had been fed with the anion regulator-added feeds, was measured. Experiments were conducted using six(6) Holstein calves with a body weight of approximately 200 kg. They were divided into three groups, two calves in a group. The groups were each fed with one of the following three(3) types of experimental feeds. Feed consisting of 1,100 kg concentrated feed ("Beef-fast", ex Itochu Feed Co.), 550 g of timothy-hay and 550 g of alfalfa hay cubes was fed, twice a day, at 9 and 16 o'clock, for one week before the start of the experiment, for adaptation.

27 g of NH₄Cl, 91 g of L-lysine monohydrochloride and 106 g of L-arginine monohydrochloride were respectively measured to be in an amount equivalent to -500 meg, and each compound was separately added to 200 g of concentrated feed. These three(3) types of mixed feeds were separately supplied to each group at 9 and 16 o'clock on the same day and at 9 o'clock on the next day. After 10 minutes, each mixed feed was removed, and subsequently, a mixture of 900 g of concentrated feed, 500 g of timothy-hay and 550 g of alfalfa hay cubes, was supplied instead. The DCAD value of the total feed prepared was approximately -150 meq/kg-dry feed matter per head, which was within the commonly recommended value range.

Measured changes in the urinary pH are shown in Table 3 and Fig. 1. In the table and figure, for example, terms Lys HCl 1 and Lys HCl 2 indicate respectively the two calves in the first group which was fed with Lys HCl (Lys HCl-feeded group).

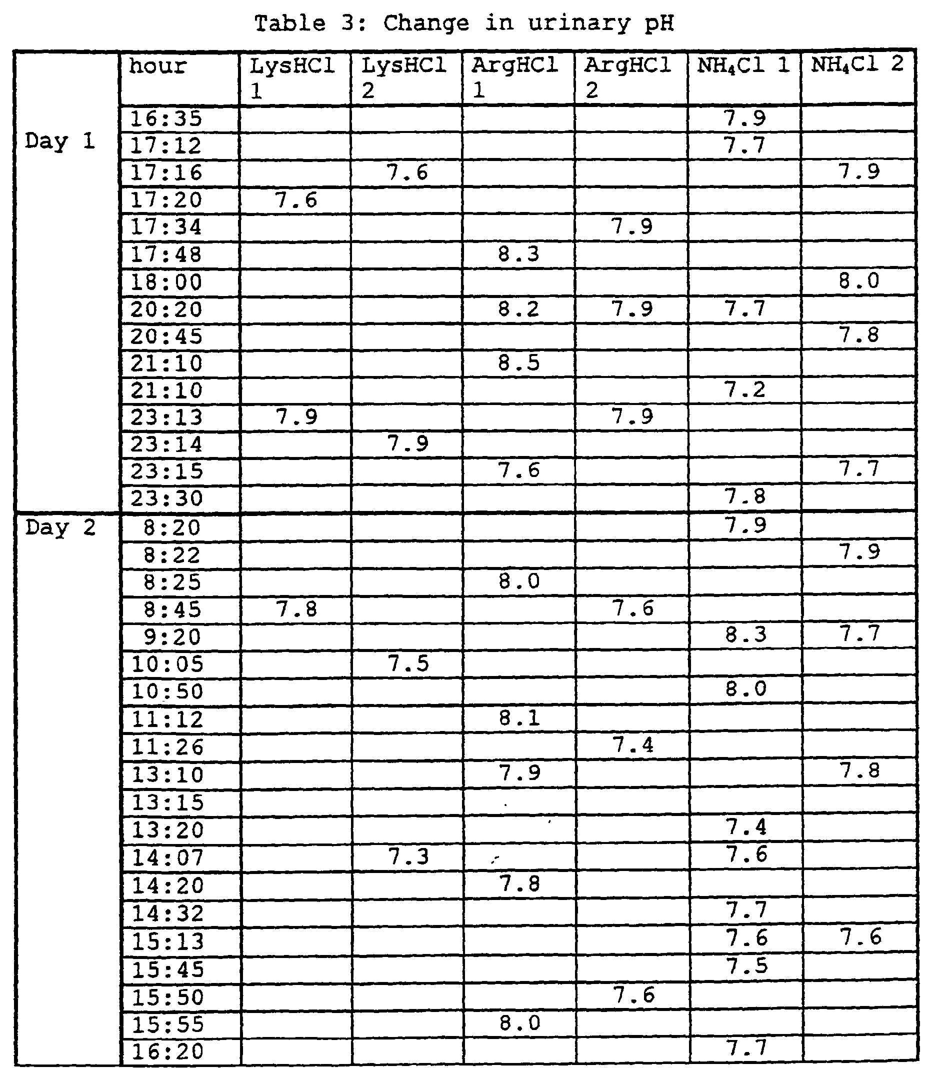

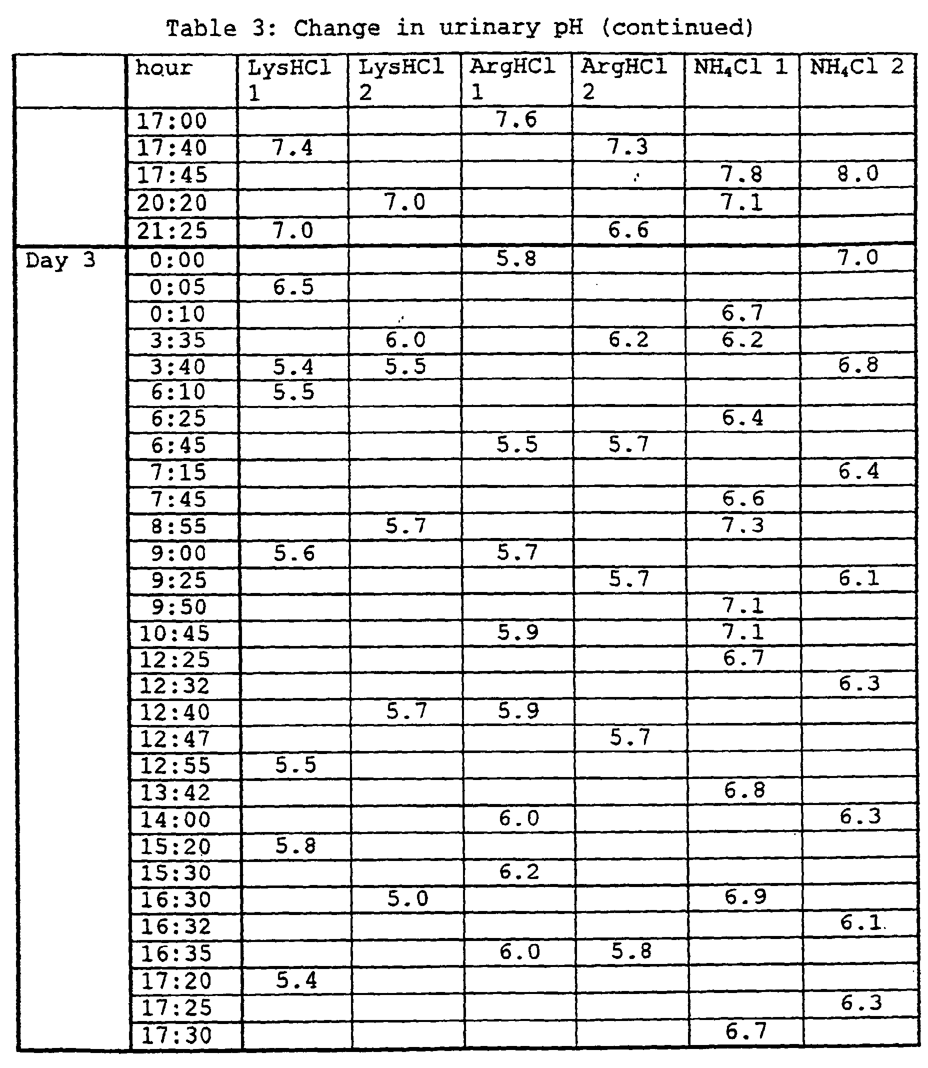

As is understood from Table 3 and Fig. 1, lysine hydrochloride (Lys HCl) and arginine hydrochloride (Arg HCl) are effective as anion regulators. Feed admixed with these compounds is eaten well. These compounds also can reduce urinary pH better than NH₄Cl, and consequently these compounds can be excellent anion regulators.

### (Industrial Applicability)

According to the present invention, anion regulators which have good palatability, which do not reduce the feed intake by mother animals before parturition, and which also show excellent anion regulating action, can be easily provided.

## Claims

1. An anion regulator for ruminants comprising the neutral hydrochloride of an amino compound as the active ingredient.

2. The anion regulator for ruminants as set forth in Claim 1 wherein the said neutral hydrochloride of an amino compound is selected from the group consisting of lysine monohydrochloride, ornithine monohydrochloride and arginine monohydrochloride.

3. A feed for ruminants comprising the anion regulator as set forth in Claim 1 or 2.
